# EUROPÄISCHE PATENTANMELDUNG

(11) **EP 0 722 789 A1**
(43) Veröffentlichungstag der Anmeldung: **24.07.1996**
(21) Anmeldenummer: 95119475.2
(22) Anmeldetag: 11.12.1995
(51) Int. Cl.: B08B 9/46, G01N 33/44, H01J 49/04, G01N 33/00

(54) **Verfahren zum Testen einer Funktion von einem Detektor in einer Inspektionsstation**

(30) Priorität: 17.01.1995 CH 130/95
(71) Anmelder: ELPATRONIC AG, CH-6303 Zug (CH)
(72) Erfinder: Robertson, Peter M., Dr., CH-8185 Winkel (CH); Rosatzin, Martin, Dr., CH-8953 Dietikon (CH)

(57) **Zusammenfassung**

Bei einem Verfahren zum Testen einer Funktion von zumindest einem Detektor (1) in einer Inspektionsstation (S) für Behälter (1), insbesondere in einer Inspektionseinrichtung (R) für Mehrwegflaschen (1), soll der Inspektionsstation bzw. dem zumindest einen Detektor in zeitlichen Abständen ein Testgas aus einer Quelle (10) zugeführt werden.

## Beschreibung

Die Erfindung betrifft ein Verfahren zum Testen einer Funktion von zumindest einem Detektor in einer Inspektionsstation für Behälter, insbesondere in einer Inspektionseinrichtung für Mehrwegflaschen, sowie eine Vorrichtung hierfür.

Insbesondere wiederverwendbare Behälter müssen heute in der Regel vor einem Wiederbefüllen nicht nur gereinigt und auf ihre Funktionsfähigkeit hin untersucht werden, sondern auch darauf, welchen Inhalt sie vor dem Wiederbefüllen enthielten. Dies gilt vor allem auch für heute in verstärkterem Umfang verwendete Mehrwegflaschen, da eine eingefüllte Fremdsubstanz die Qualität der Mehrwegflasche, insbesondere der PET-Flasche, erheblich beeinflussen kann. Hierzu werden heute Inspektionseinrichtungen, z.B. gemäss EP 0579055 oder EP 0579952 oder EP 0578146, eingesetzt, welche die zur Wiederverwertung vorgesehenen Behälter auf eine Vielzahl von Kriterien hin untersuchen. Entspricht dabei ein Behälter nicht den gewünschten Anforderungen, so wird er vor dem Waschen und Wiederbefüllen ausgeschieden und in der Regel vernichtet.

Diesen Inspektionseinrichtungen kommt somit in zunehmendem Masse eine immer grösser werdende Bedeutung zu. Voraussetzung ist dabei, dass die Inspektionseinrichtung absolut zuverlässig arbeitet und sehr empfindlich auf Fremdsubstanzen reagiert.

Als Fremdsubstanz müssen unter anderem schwerflüchtige Bestandteile von Benzin und anderen Kohlenwasserstoff-Gemischen, also z.B. auch polyaromatische Kohlenwasserstoffe, erkannt werden. Für deren Detektion werden spezifische Detektoren eingesetzt. Während des normalen Produktionsbetriebes muss aber Online die Funktionstüchtigkeit dieser Detektoren mit künstlich kontaminierten Behältern (= Testbehälter) periodisch getestet werden. Die Zuverlässigkeit dieses Testes ist wiederum unmittelbar von der Reproduzierbarkeit der Testbehälter abhängig. Gerade aber die Herstellung von mit polyaromatischen Kohlenwasserstoffen kontaminierten Testbehältern bereitet erhebliche Schwierigkeiten. Sie sollen ungiftig sein, reproduzierbar, zuverlässig und einfach herzustellen.

Als bislang einziges Verfahren werden deshalb in entsprechende Testbehälter einige Tropfen eines polyaromatischen Kohlenwasserstoffes gegeben oder dieser in einem Lösungsmittel aufgelöst. Daraus ergeben sich jedoch verschiedene Unzulänglichkeiten. Wegen der notwendigen extremen Empfindlichkeit der Detektoren für die polyaromatischen Kohlenwasserstoffe ist auch bei einer Verdünnung der polyaromatischen Kohlenwasserstoffe in einem Lösungsmittel die in einen Testbehälter einzubringende Menge so gering (typischerweise einige µl), dass für eine entsprechende Dosierung aufwendige Dosiersysteme und geschultes Personal erforderlich sind.

Darüberhinaus ist die Reproduzierbarkeit wegen der schwierigen Handhabung nicht ohne weiteres gegeben. Ferner sind die verwendbaren Lösungsmittel für die polyaromatischen Kohlenwasserstoffe im allgemeinen leicht flüchtig oder giftig. Wegen dem hohen Dampfdruck bei leicht flüchtigen Lösungsmitteln, sprechen auf diese in den Inspektionsein-richtungen eingesetzte, komplementäre Detektionsgeräte, wie beispielsweise ein Massenspektrometer, an, so dass bereits die entsprechenden Behälter wegen dieser Lösungmittel ausgeschieden werden und ein direkter Test des eigentlichen Detektors für die polyaromatischen Kohlenwasserstoffe unmöglich wird.

Giftige Lösungsmittel müssen für den Lebensmittelbereich von vorne herein ausgeschlossen werden.

Im vorliegenden Fall bezieht sich die Beschreibung vor allem auf ein Verfahren zum Befüllen von Mehrwegflaschen mit einer Testsubstanz. Im Rahmen der Erfindung liegt jedoch das Befüllen jedes beliebigen Testbehälters mit einer entsprechenden Testsubstanz zu einem beliebigen Zweck.

Der vorliegenden Erfindung liegt die Aufgabe zugrunde, ein Verfahren und eine Vorrichtung der o.g. Art zu entwickeln, mittels welchen auf einfache Art und Weise die Prüfung der Funktionsfähigkeit von entsprechenden Detektoren erfolgt.

Zur Lösung dieser Aufgabe führt, daß der Inspektionsstation bzw. dem zumindest einen Detektor in zeitlichen Abständen ein Testgas aus einer Quelle zugeführt wird.

Damit entfällt die Gefahr einer Querbeeinflussung anderer Detektoren durch ein Lösungsmittel. Dieses eine Testgas spricht tatsächlich nur den Detektor an, der für sie bestimmt ist. Wird zum Beispiel Naphthalin als kritischer Stoff mit Toluol als Lösungsmittel versetzt und in einen Testbehälter eingebracht, so wird dieser Testbehälter bereits wegen des Toluols durch einen auf diese Substanz ansprechenden Massenspektrometer ausgeschieden. Der eigentliche Detektor für das Naphthalin kann nicht ohne zusätzlichen Aufwand auf seine Funktionsfähigkeit hin geprüft werden.

Ein weiterer wesentlicher Vorteil der vorliegenden Erfindung liegt jedoch darin, dass ein Testgas in sehr geringer Konzentration dem Detektor direkt zugeführt oder in einen Testbehälter eingebracht werden kann. Hierzu ist erfindungsgemäss vorgesehen, dass das Testgas vor dem Zuführen zum Detektor oder dem Einbringen in den Testbehälter in zumindest einer ersten Verdünnungsstufe mit einem anderen Gas verdünnt und, bei Verwendung eines Testbehälters, in einer zweiten Verdünnungsstufe in dem Testbehälter selbst mit dem darin vorhandenen Gas, in der Regel Luft, nochmals verdünnt wird. Dies hat zur Folge, dass der Testbehälter mit einem Testgas in einer derartigen Konzentration befüllt ist, wie dies durch eine Testflüssigkeit kaum möglich ist. Gleichzeitig erfolgt aber durch die geringe Konzentration eine sehr gute Bestimmung der Funktionstüchtigkeit des Detektors in der Inspektionseinrichtung.

Bevorzugt wird das Testgas aus einer Flüssigkeit durch Verdampfen oder aus einem Festkörper durch Sublimation erzeugt. Da die Menge der Moleküle in der Fest- bzw. Flüssigphase um typischerweise sechs Grössenordnungen grösser ist, als jene in der Gasphase, ist eine extrem gute Langzeitstabilität der Konzentration in der Gasphase gewährleistet. Da zudem das Testgasvolumen in der ersten Verdünnungsstufe sehr viel grösser ist als das Volumen, das anschliessend bevorzugt mit einer Dosierpumpe entommen wird, ist auch eine gute Reproduzierbarkeit der Konzentration des verdünnten Testgases gegeben. Gerade diese Regulierung bzw. Dosierung des Testgasvolumens wird bei einer bevorzugten Ausführungsform des Verfahrens durchgeführt.

Es ist bekannt, dass sich die Konzentration in einem Testgasvolumen, welches durch Verdampfen aus einer Flüssigkeit oder durch Sublimation aus Festkörpern entsteht, temperaturabhängig ist. Gewünscht wird aber immer die gleiche Menge an Testgas, die dem Detektor zugeführt oder dem Testbehälter eingegeben wird. Bei Trennung des erfindungsgemässen Verfahrens in zwei Verdünnungsstufen kann eine derartige Dosierung bzw. Regulierung auf einfache Art und Weise beispielsweise durch eine Dosierpumpe erfolgen. Dabei soll in einem bevorzugten Ausführungsbeispiel dieser Dosierpumpe ein Temperatursensor zugeordnet sein, an dem gleichzeitig die Einstellung der Dosierpumpe ablesbar ist. Es versteht sich von selbst, dass die Einstellung der Dosierpumpe auch auf automatischem Wege erfolgen kann, indem in einer entsprechenden Einrichtung die Regulierung der Dosierpumpe mit einem Temperatursensor verbunden ist, der anhand einer vorgegebenen Kurve die Dosierpumpe einstellt. Möglich ist auch die Verwendung eines Temperaturmessstreifens als Skala zur Einstellung des Pumphubs. Dadurch entfällt eine Konvertierung der Temperatur durch den Anwender.

Damit zudem eine gute Reproduzierbarkeit der Testbehälter möglich ist, sollte in dem Testbehälter sowenig wie möglich an Testgas verloren gehen. Dies geschieht dadurch, dass der Testbehälter mit einer gasundurchlässigen Innenbeschichtung versehen ist.

Im Rahmen der vorliegenden Erfindung liegt auch eine Vorrichtung zum Testen einer Funktion von zumindest einem Detektor in einer Inspektionsstation für Behälter, insbesondere in einer Inspektionseinrichtung für Mehrwegflaschen, wobei mit dem Detektor eine Quelle für ein Testgas oder ein ein Testgas aus einer Quelle beinhaltender Testbehälter verbindbar ist. In einem einfachen Ausführungsbeispiel der Erfindung kann die Quelle bereits ein vorgegebener Druckbehälter sein, der ein gewünschtes, verdünntes Testgasvolumen beinhaltet. Dies wird dann entsprechend über eine Dosierpumpe geregelt dem Detektor direkt zugeführt oder in den Testbehälter eingefüllt.

Da jedoch die Vorrichtung so einfach wie möglich ausgestaltet sein sollte, besteht die Quelle in dem bevorzugten Ausführungsbeispiel aus einer Flasche, in welcher sich ein Filter zum Abtrennen einer unteren und einer oberen Kammer befindet. In der unteren Kammer befindet sich eine Flüssigkeit oder aber bevorzugt Festkörper, aus denen durch Sublimation das entsprechende Testgas in die obere Kammer entweicht. Der Filter verhindert dabei eine Vermischung von Feststoffpartikeln mit dem Gas, wodurch eine saubere Trennung von Festkörper und Gas möglich ist. Ferner kann durch den Filter der Festkörperanteil nicht versehentlich ausgeleert werden.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 einen teilweise schematisch dargestellten Ausschnitt aus einer Inspektionseinrichtung zum Inspizieren von wiederbefüllbaren Behältern;
Figur 2 eine Ansicht einer erfindungsgemäße Vorrichtung zum Befüllen eines Testbehälters mit einem Testgas;
Figur 3 eine Draufsicht auf einen Temperatursensor bzw. - messstreifen.

In einer ansonsten nicht näher gezeigten Inspektionseinrichtung R zum Inspizieren von wiederbefüllbaren Behältern 1 werden diese Behälter 1 durch eine Zuführeinrichtung 2, beispielsweise ein entsprechendes Transportband, einer Inspektionsstation S zugeführt. In dieser Inspektionsstation S können die Behälter auf verschiedenste Kriterien hin untersucht werden. Hierzu gehört neben Kriterien, die den Behälter selbst betreffen, wie beispielsweise der Zustand eines Gewindes, der Aussenwand usw., die Untersuchung über einen möglichen vorherigen Inhalt des Behälters, der diesen Behälter unbrauchbar macht oder aber einen bestimmten Waschvorgang erfordert.

Zur Bestimmung eines unerwünschten Inhaltes wird dem Behälter 1 durch ein Saugrohr 3 eine Probe entnommen und über eine Leitung 4 einem Massenspektrometer 5 zugeführt. In diesem Massenspektrometer 5 kann sowohl eine quantitative Analyse von Gasen und Flüssigkeiten als auch eine qualitative Analyse von anorganischen und organischen Verbindungen erfolgen, woraus auf den vorherigen Inhalt des Behälters geschlossen werden kann.

In einem wesentlichen Ausführungsbeispiel der Erfindung wird die Probe zusätzlich über eine Leitung 7 einem Pulsfluoreszenz- Detektor 8 zur Bestimmung von polyaromatischen Kohlenwasserstoffen zugeführt. Diese polyaromatischen Kohlenwasserstoffe finden sich vor allem in Benzin, so dass aus ihrer Bestimmung sehr leicht erkannt werden kann, ob in dem Behälter, beispielsweise einer Trinkflasche, vor der Rückgabe Benzin enthalten war.

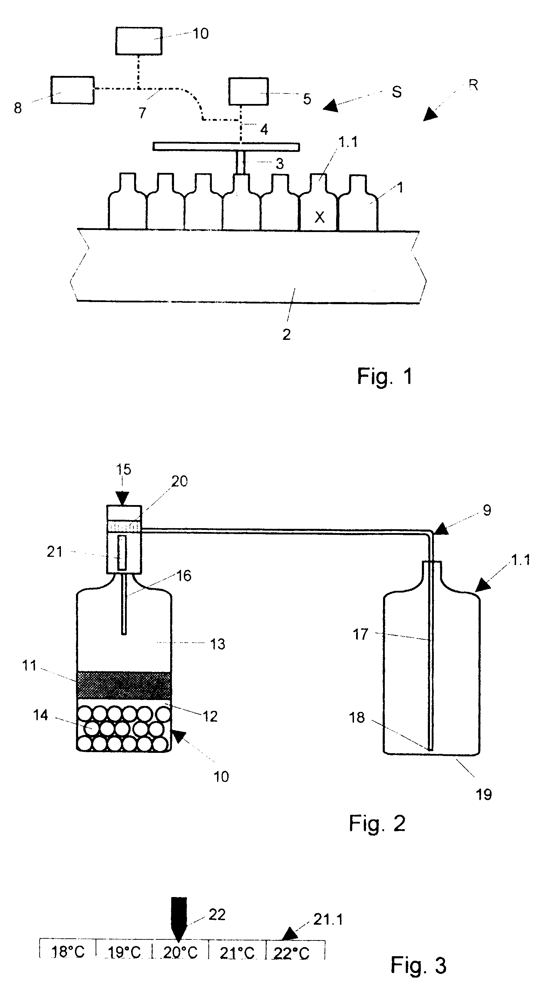

Von Zeit zu Zeit muß jedoch auch die Inspektionsstation S auf ihre Funktionsfähigkeiten hin überprüft werden. Hierzu sind zwei Wege in den Figuren 1 und 2 angedeutet. In jedem Fall wird dem Detektor 8 ein Testgas, möglichst in grosser Verdünnung zugeführt. Dies kann einmal dadurch geschehen, dass dieses Testgas auf Wunsch bzw. in zeitlichen Abständen direkt aus einer Quelle 10 in die Leitung 7 eingespeist oder sonstwie dem Detektor zugeführt wird. Ferner können aber auch zwischen die einzelnen Behälter 1 Testflaschen 1.1 eingeschleust werden, die mit einem entsprechenden unerwünschten Inhalt kontaminiert wurden.

Die vorliegende Erfindung bezieht sich vor allem auf eine Befüllung eines derartigen Testbehältes 1.1 mit diesem unerwünschten Inhalt gemäss Figur 2. Hierzu steht der Testbehälter 1.1 gemäss Figur 2 über einer Leitung 9 mit einer Quelle 10 für ein Testgas in Verbindung. Im vorliegenden Ausführungsbeispiel ist die Quelle 10 als eine Reservoir-Flasche ausgestaltet.

Die Quelle 10 ist bevorzugt durch eine Separator 11 in eine untere Kammer 12 und eine obere Kammer 13 unterteilt. Dabei kann es sich bei diesem Separator der Einfachheit halber um einen mechanischen Separator, wie beispielsweise einen Filter, handeln.

In die untere Kammer 12 sind Festkörper 14, beispielsweise aus Naphthalin, eingefüllt. In der oberen Kammer befindet sich ein Gasvolumen, beispielsweise Luft. Durch Sublimation gelangt ein Testgas in stark verdünnter Form durch den Separator 11 in die obere Kammer 13, vermischt sich dort mit dem Gasvolumen und bildet einen Dampf. Im Gleichgewicht zwischen der Gasphase und dem Festkörper ist die Konzentration des Testgases nur abhängig von der Temperatur. Dies ist die erste Verdünnungsstufe für das Testgas.

Über eine Dosierpumpe 15 wird durch ein Ansaugrohr 16 ein gewünschtes, definiertes Volumen aus der oberen Kammer 13 entnommen und dieses dem Testbehälter 1.1 über die Leitung 9 zugeführt. Dabei weist die Leitung 9 eine Stichleitung 17 auf, die so tief in den Testbehälter 1.1 eingeschoben ist, dass sich ihre Mündung 18 kurz oberhalb eines Bodens 19 des Testbehälters 1.1 befindet. Hierdurch verbleibt das gewünschte Testgasvolumen in dem Testbehälter 1.1, während die Stichleitung 17 ohne zusätzliche unkontrolliert Verdünnung des von der Quelle in den Testbehälter gepumpten Testgasvolumens aus dem Testbehälter 1.1 herausgezogen und der Testbehälter ggfs. verschlossen werden kann.

Da sich jedoch in dem Testbehälter ein Gas, in der Regel Luft, befindet, wird das von der Quelle 10 überführte Testgasvolumen nochmals in einer zweiten Verdünnungsstufe verdünnt. Hierdurch wird eine sehr niedrige Konzentration an Testgasvolumen in dem Testbehälter 1.1 erreicht, die gerade wünschenswert ist, um den Detektor 8 auf seine Funktionsfähigkeit hin zu überprüfen. Beispielsweise beträgt die Verdünnung in der ersten Verdünnunsstufe für Naphthalin vom Festkörper zur Gasphase bei 20°C ca. 10⁶. Die Verdünnung in der zweiten Stufe beträgt nochmals etwa 100, wodurch eine totale Verdünnung von 10⁸ erreicht wird.

In der Regel verläuft eine Sublimation, d.h., eine direkte Überführung eines Feststoffes in einen gasförmigen Zustand ohne vorübergehende Verflüssigung temperaturabhängig. Damit ist aber auch die Konzentration des Testgasvolumen in der oberen Kammer 13 von der jeweiligen Temperatur abhängig, so dass Testbehältern 1.1 durch die Dosierpumpe 15, sofern diese nicht eingestellt werden kann, unterschiedliche Mengen an Testgas zugeführt werden könnten. Um dies auszuschliessen, soll erfindungsgemäss die Dosierpumpe 15 eingestellt bzw reguliert werden können. Dabei ist der Einfachheit halber vorgesehen, dass auf der Dosierpumpe ein Temperaturmessstreifen 21 angeordnet ist, an dem die Temperatur abgelesen werden kann. Gleichzeitig gibt dieser Messstreifen 21 auch eine Angabe darüber, wie die Dosierpumpe einzustellen ist.

Eine Skala 20 zur Einstellung der Dosierpumpe 15 ist ebenfalls angedeutet. Durch Drehen dieser Skala 20 kann beispielsweise ein Pumphub der Dosierpumpe 15 vergrössert oder verkleinert werden. Hierdurch wird das entsprechende Fördervolumen an Testgas durch die Leitung 9 in den Testbehälter 1.1 definiert. Die zweite Verdünnungsstufe in dem Testbehälter 1.1 kompensiert damit die Temperaturabhängigkeit der ersten Verdünnungsstufe in der oberen Kammer 13 und macht somit beide Verdünnungsstufen unabhängig von einer Temperatur bzw. einer Temperaturänderung. Ein Beschicken der Testbehälter 1.1 mit einem definierten Testgasvolumen wird somit reproduzierbar.

Der Temperaturmessstreifen 21.1 kann gemäss Figur 3 auch so ausgestaltet sein, dass er direkt als Einstellskala 20 für das Pumpvolumen dient. Ein Zeiger 22 der Dosierpumpe 15 braucht dann nur auf die entsprechende Temperatur gestellt zu werden.

Nachdem der Testbehälter 1.1 befüllt, die Stichleitung 17 herausgezogen und der Testbehälter 1.1 verschlossen worden ist, verteilt sich das Testgas als Dampf gleichmässig auf das gesamte Volumen des Testbehälters. Infolge der extremen Verdünnung in den beiden Verdünnungsstufen liegt die Konzentration eines Testgases in dem Testbehälter deutlich tiefer als die Sättigungskonzentration des Testgases bei der bestehenden Temperatur. Dadurch findet keine empfindliche Reaktion zwischen Testgas und Testbehälter 1.1 auf grössere Temperaturunterschiede zwischen beiden Behältern statt, d.h., es kommt nicht zu einer Kondensation des Testgases in dem Testbehälter 1.1, was ansonsten die Reproduzierbarkeit beeinträchtigen würde.

## Patentansprüche

1. Verfahren zum Testen einer Funktion von zumindest einem Detektor (8) in einer Inspektionsstation (S) für Behälter (1), insbesondere in einer Inspektionseinrichtung (R) für Mehrwegflaschen (1),
dadurch gekennzeichnet,
dass der Inspektionsstation (S) bzw. dem zumindest einen Detektor (8) in zeitlichen Abständen ein Testgas aus einer Quelle (10) zugeführt wird.

2. Verfahren nach Anspruch 1, dadurch gekennzeichnet, dass das Testgas direkt aus der Quelle (10) eine Leitung (7) zum Detektor (8) eingespeist wird.

3. Verfahren nach Anspruch 1, dadurch gekennzeichnet, dass das Testgas aus der Quelle (10) in einen Testbehälter (1.1) eingegeben und dieser unter die anderen Behälter (1) gemischt wird.

4. Verfahren nach einem der Ansprüche 1 bis 3, dadurch gekennzeichnet, dass das Testgas in der Quelle (10) aus einer Flüssigkeit durch Verdampfen oder aus einem Testkörper durch Sublimation erzeugt wird.

5. Verfahren nach Anspruch 4, dadurch gekennzeichnet, dass das Testgas in einer ersten Verdünnungsstufe in der Quelle (10) mit einem anderen Gas verdünnt wird.

6. Verfahren nach Anspruch 5, dadurch gekennzeichnet, dass das aus der ersten Verdünnungsstufe in den Testbehälter (1.1) bzw. dem Detektor (8) einzubringende Testgasvolumen dosiert bzw. reguliert wird.

7. Verfahren nach Anspruch 6, dadurch gekennzeichnet, dass das in den Testbehälter (1.1) bzw. den Detektor (8) einzubringende Testgasvolumen temperaturabhängig bestimmt wird.

8. Verfahren nach einem der Ansprüche 5 bis 7, dadurch gekennzeichnet, dass das Testgas nach der ersten Verdünnungsstufe in einer zweiten Verdünnungsstufe in dem Testbehälter (1.1) selbst mit dem darin vorhandenen Gas nochmals verdünnt wird.

9. Verfahren nach einem der Ansprüche 3 bis 8, dadurch gekennzeichnet, dass der Testbehälter (1.1) mit einer gasundurchlässigen Innenbeschichtung versehen wird.

10. Vorrichtung zum Testen einer Funktion von zumindest einem Detektor (8) in einer Inspektionsstation (S) für Behälter (1), insbesondere in einer Inspektionseinrichtung (R) für Mehrwegflaschen (1), dadurch gekennzeichnet, dass mit dem Detektor (8) eine Quelle (10) für ein Testgas direkt verbindbar ist.

11. Vorrichtung zum Testen einer Funktion von zumindest einem Detektor (8) in einer Inspektionsstation (S) für Behälter (1), insbesondere in einer Inspektionseinrichtung (R) für Mehrwegflaschen (1), dadurch gekennzeichnet, dass mit dem Detektor (8) ein ein Testgas aus einer Quelle (10) beinhaltender Testbehälter (1.1) verbindbar ist.

12. Vorrichtung nach Anspruch 10 oder 11, dadurch gekennzeichnet, dass die Quelle (10) für das Testgas eine ein Testgasvolumen beinhaltenden Kammer (13) aufweist.

13. Vorrichtung nach Anspruch 12, dadurch gekennzeichnet, dass zwischen der Kammer (13) eine Dosiereinrichtung (15) für das aus der Kammer (13) zu entnehmende Testgasvolumen nachgeschaltet ist.

14. Vorrichtung nach Anspruch 13, dadurch gekennzeichnet, dass die Dosiereinrichtung (15), insbesondere eine Dosierpumpe, zum Ausgleich von temperaturabhängigen Konzentrationsschwankungen mit einem Temperatursensor (21) gekoppelt ist.

15. Vorrichtung nach Anspruch 14, dadurch gekennzeichnet, dass der Temperatursensor (21) ein Temperaturmessstreifen (21.1) ist, der auch als Einstellskala (20) für die Dosiereinrichtung (15) dient.

16. Vorrichtung nach einem der Ansprüche 10 bis 15, dadurch gekennzeichnet, dass der Kammer (13) eine weitere Kammer (12) zur Aufnahme einer das Testgas erzeugenden Flüssigkeit oder einem Festkörper (14) zugeordnet ist.

17. Vorrichtung nach Anspruch 16, dadurch gekennzeichnet, dass die beiden Kammern (12 und 13) durch einen Separator (11), insbesondere einen Filter, voneinander getrennt sind.

18. Vorrichtung nach einem der Ansprüche 11 bis 17, dadurch gekennzeichnet, dass aus der Kammer (13) eine Leitung (9) zum Einbringen des Testgasvolumens in den Testbehälter (1.1) ausmündet.
